(19) Europäisches Patentamt
European Patent Office
Office européen des brevets

(11) Veröffentlichungsnummer: **0 007 513**
**B1**

(12) # EUROPÄISCHE PATENTSCHRIFT

(45) Veröffentlichungstag der Patentschrift: **19.05.82**

(51) Int. Cl.$^3$: **C 07 C 45/39**, C 07 C 49/12

(21) Anmeldenummer: **79102364.1**

(22) Anmeldetag: **10.07.79**

(54) **Verfahren zur Herstellung von Diketonen.**

(30) Priorität: **15.07.78 DE 2831229**

(43) Veröffentlichungstag der Anmeldung:
**06.02.80 Patentblatt 80/3**

(45) Bekanntmachung des Hinweises auf die Patenterteilung:
**19.05.82 Patentblatt 82/20**

(84) Benannte Vertragsstaaten:
**BE CH DE FR GB NL**

(56) Entgegenhaltungen:
**DE - C - 854 795**
**US - A - 2 051 266**

(73) Patentinhaber: **BASF Aktiengesellschaft**
**Carl-Bosch-Strasse 38**
**D-6700 Ludwigshafen (DE)**

(72) Erfinder: **Dudeck, Christian, Dr.-Chem.**
**Odenwaldring 20**
**D-6703 Limburgerhof (DE)**
Erfinder: **Lehmann, Gunter, Chem. Ing.**
**Bayernstrasse 58**
**D-6700 Ludwigshafen (DE)**
Erfinder: **Ross, Karl-Heinz, Dr.-Chem.**
**Sudetenstrasse 8**
**D-6704 Mutterstadt (DE)**
Erfinder: **Fliege, Werner, Dr.-Chem.**
**c/o BASF Wyandotte Corp.Central R+D P.O.Box 111**
**Wyandotte, Mich-48192 (US)**
Erfinder: **Petri, Norbert, Dr.-Chem.**
**Max-Beckmanmn-Strasse 17**
**D-6710 Frankenthal (DE)**
Erfinder: **Diem, Hans, Dr.-Chem.**
**Feldbergstrasse 63**
**D-6800 Mannheim 25 (DE)**
Erfinder: **Meissner, Bernd, Dr.-Chem.**
**Dammweg 15**
**D-6900 Heidelberg 1 (DE)**
Erfinder: **Sauer, Wolfgang, Dr.-Chem.**
**Kaefertaler Strasse 183**
**D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von Diketonen

Die Erfindung betrifft ein Verfahren zur Herstellung von Diketonen durch Oxidation von Glykolen in Gegenwart eines Silber- und/oder Kupferkatalysators bestimmter Korngröße und unter bestimmten Bedingungen der Temperatur und Katalysatorzusammensetzung.

Es ist aus Ullmanns Encyklopädie der technischen Chemie, Band 8, Seiten 250 bis 252, bekannt, daß eines de wichtigsten Verfahren zur Herstellung von Glyoxal die Oxidation von Äthylenglykol mit Luft ist. Als Bedingungen werden Temperaturen von 300 bis 325°C, Kupferoxid als Katalysator und der Zusatz von Halogenverbindungen genannt. Man erhält aus dem Reaktionsgemisch durch Absorption in einer Glyoxallösung oder in Wasser eine 32-prozentige Glyoxallösung. Ein solches Verfahren liefert Ausbeuten von höchstens 65 Prozent und Raum-Zeit-Ausbeuten von nur 0,04 bis 1,5 Gramm Glyoxal/$cm^3$ Katalysatorvolumen und Stunde.

Es ist aus Proceed. Acad. Sci. USSR, Chem. Ser. (1964), Seiten 641 bis 643, bekannt, Silber als Katalysator auf Bimsstein und Aluminium als Träger zu verwenden. Es wird festgestellt, daß mit solchen Katalysatoren nur Schlechte Ergebnisse erzielt werden. Daher verwendet man Silberspiralen als Katalysatoren. Das beste Ergebnis wurde mit Silberspiralen einer Temperatur von 600°C, einem Druck von 544 bis 816 mbar und einer Ausbeute von 69 Prozent erhalten. Der Endstoff fällt in Gestalt einer 25-gewichtsprozentigen, wäßrigen Lösung mit zusätzlich 5 bis 10 Prozent Glykol an. Aus den mitgeteilten Daten ergibt sich eine Verweilzeit von 0,037 Sekunden oder eine Raum-Zeit-Ausbeute von 4,46 Gramm Glyoxal pro Stunde und $cm^3$ Katalysatorvolumen. Zusätzliches Inertgas wird nicht verwendet. Die Arbeitsweise mit Unterdruck ist auch im Hinblick auf die Raum-Zeit-Ausbeute unbefriedigend.

Die russische Patentschrift 136 352 beschreibt die Oxidation von Glykol bei 500 bis 700°C, wobei als Katalysator Silber auf Aluminiumoxid (40% Ag) verwendet wird. Der Katalysator wird vor der Verwendung bei 600 bis 700°C während 2 Stunden geglüht. Die Strömungsgeschwindigkeit beträgt 2,1 Meter pro Sekunde. Die Ausgangsmischung enthält 40 Prozent Glykol und 60 Prozent Wasser. Die Ausbeute beträgt 61 Prozent, die Raum-Zeit-Ausbeute 12,8 Gramm Glyoxal pro Stunde und Gramm Katalysator. Das Verfahren hat den Nachteil, daß der Katalysator umständlich herzustellen ist und eine ziemlich verdünnte Glykollösung eingesetzt werden muß. Wenn der Katalysator verbraucht ist, z.B. durch Vergiftung, muß er in zahlreichen chemischen Operationen aufgearbeitet werden.

In der deutschen Auslegeschrift 1 032 732 wird dargelegt, daß man bei Verwendung von Kupfer und Silber als Katalysator einen Promotor, z.B. $TiO_2$ und $Mo_2O_5$, benötigt und zur Erhöhung der Ausbeute Hemmstoffe, z.B. HCl, $Cl_2$ oder Äthylendichlorid zusetzen muß. Als bestes Ergebnis erhält man danach eine Raum-Zeit-Ausbeute von 0,043 Gramm pro $cm^3$ und Stunde. Man kann nach der Lehre der Auslegeschrift das Ergebnis verbessern, wenn man das Silber auf Bimsstein, Silicagel oder Aluminiumoxid als Träger aufbringt. Das Verfahren wird zwischen 300 und 450°C unter Verwendung eines Luft-Stickstoffgemisches mit einem Sauerstoffgehalt zwischen 1,6 und 5 Prozent durchgeführt. Bei einer Ausbeute von 55 Prozent wird eine Raum-Zeit-Ausbeute von 0,104 Gramm Glyoxal pro $cm^3$ Katalysatorraum und Stunde erreicht. Die Raum-Zeit-Ausbeute ist unbefriedigend.

Die deutsche Offenlegungsschrift 1 923 048 beschreibt die Herstellung von Glyoxal und verwendet als Katalysator zwei Komponenten (a und b) und zwar.

a) Kupfer oder Silber und/oder Gold und außerdem

b) Germanium, Zinn, Blei, Stickstoff, Phosphor, Arsen, Antimon und/oder Wismut.

Bevorzugt ist Silber in Verbindung mit Zinn, Phosphor und/oder Arsen und insgesamt insbesondere Kupfer gegenüber Silber. Eine Reaktionstemperatur von ca. 180 bis 600°C, vorzugsweise von ca. 300 bis 450°C, wird angegeben. Verdünnungsgase mit vorzugsweise einem Molverhältnis von Verdünnungsgas zu Sauerstoff im Bereich von 5 bis 200 zu 1 können verwendet werden. Geeignete Verweilzeiten liegen zwischen 0,1 und 20 Sekunden, wobei solche von 1 bis 5 Sekunden bevorzugt sind. Das vergleichsweise zu zahlreichen Beispielen mit Kupferkatalysatoren einzige Beispiel mit einem Kupferfreien Silberkatalysator (Silber/Phosphor) wird bei einer Temperatur von ca. 430 bis 450°C durchgeführt. Aus den Angaben errechnet sich eine unbefriedigende Raum-Zeit-Ausbeute. Nachteilig ist ferner die umständliche Katalysatorstellung.

In der deutschen Offenlegungsschrift 2 634 439 wird ein Katalysator verwendet, der aus Phosphor kombiniert mit Cu und/oder Ag besteht. Der Reaktion wird eine Bromverbindung zugesetzt, die dazu ausreicht, die Glyoxalausbeute zu erhöhen, die jedoch nicht so groß ist, daß die Glykolaldehydbildung merklich gesteigert wird oder die Umwandlung des Äthylenglykols auf weniger als etwa 90 Prozent absinkt. Inertgas wird zugesetzt. In den Beispielen wird stets ein Kupfer/Silber/Phosphor-Katalysator verwendet. Die Raum-Zeit-Ausbeute beträgt nur 1,5 Gramm Glyoxal pro $cm^3$ Katalysator und Stunde. Ein weiterer Nachteil ist, daß der Katalysator nur umständlich regeneriert werden kann.

Es ist aus den Annalen der Chemie, Band 596 (1955), Seite 160 bekannt, daß man n-Hexandiol-2,5 mit Hydrierungskatalysatoren auf etwa 150°C erhitzt und so unter Abspaltung von Wasserstoff bis 70 Prozent Hexanol-(2)-on-(5), bei höheren Temperaturen bis zu 80 Prozent Acetonylaceton erhält.

Es ist aus Tetrahedron Letters, Band 41 (1964), Seite 3 074 bekannt, daß 2,5-Hexandiol mit Bleitetraacetat in Pyridin bei Raumtemperatur in 89-prozentiger Ausbeute zu 2,5-Hexandion umgewandelt wird; die Ausbeute wird durch Erhöhung der Temperatur nicht verbessert. Essigsäureester und

unumgesetzter Alkohol treten als Nebenprodukte auf. Reaktionszeiten von 10 bis 20 Stunden sind erforderlich; die Nebenprodukte erschweren die Aufarbeitung.

Die DE—C—854 795 beschreibt ein Verfahren zur Herstellung von Diketonen und Ketoalkoholen durch Erhitzen von Glykolen auf 150 bis 400°C in Gegenwart von reinem Kupfer. Es handelt sich hierbei nicht um eine Oxidation mit Sauerstoff, sonderm um eine reine Dehydrierung. Weder in der Beschreibung, noch in dem Beispiel, noch in dem entgegengehaltenen Patentanspruch ist von einer Oxidation bzw. der Gegenwart von Sauerstoff die Rede.

Die US—S—2 051 266 beschreibt ein Verfahren zur katalytischen Oxidation von Polyalkoholen mit Sauerstoff im Temperaturbereich von 200 bis 500°C. Es werden sehr viele Di- und Triole als mögliche Ausgangsverbindungen aufgeführt.

Als Auswahl der für die Oxidation geeigneten Katalysatoren wird eine Liste von immerhin 26 verschiedenen Metalle, Metallegierungen, Oxiden un Salzen angegeben (S. 3, linke Spalte, Zeilen 59—68). Als besonders geeignet werden dabei Kupfer, Kupferoxid und Silber beschrieben. Die Herstellung der Katalysatoren kann—nach der allgemeinen Beschreibung—nach vielen Methoden geschehen; die Katalysatoren können in vielen mechanischen Formen verwendet werden (S. 3, rechte Spalte, Zeilen 13—42). Auch die Ausführung der Oxidation kann in der Flüssigphase oder in einem Gemisch von Flüssigund Gasphase oder in der Gasphase (Seite 2, rechte Spalte, Zeilen (10—12) durchgeführt werden. Das einzige Patentbeispiel liefert nur sehr dürftige Angaben (Seite 3, rechte Spalte, Zeilen 51—68). Wesentliche Versuchsbedingungen sind nicht angegeben, z.B. fehlen folgende Angaben, die auch nicht ableitbar sind: Art des Kupferkatalysators (mechan. Form u.a.), Art der Aktivierung des Katalysators (Temperatur u.a.), Menge des Katalysators (Volumen, Gewicht), Zulaufmenge an 2,3-Butylenglykol, Katalysatorbelastung, Verweilzeit Beschriebung der Versuchsanordnung.

Selbst Umsatz, Selektivität oder Ausbeute sind nicht angegeben oder zu ermitteln. Es wird zwar die Zusammensetzung des flüssigen Reaktionsaustrags angegeben; zur Berechnung einer Ausbeute fehlt aber neben der Zulaufmenge die Menge dieses Austrages und außerdem Menge und Zusammensetzung der vermutlich auftretenden gasförmigen Nebenprodukte wie CO, $CO_2$ u.a. Es wird angegeben, daß die relative Ausbeute an Ketol (Acetoin) und Diacetyl auf den Bedingungen der Versuchsdurchführung beruhe (Seite 3, rechte Spalte, Zeilen 64—66). Über diese Bedingungen und über die Ausbeuten wird aber nichts gesagt. Zusammenfassend ist unklar, welche Maßnahme man nach der Lehre dieser Schrift treffen muß, um die gewünschte Oxidation der Polyole durchzuführen und zu welchem Ergebnis (Ausbeute) dies führen muß.

Alle diese Verfahren sind im Hinblick auf einfachen und wirtschaftlichen Betrieb, einfache Katalysatorherstellung und gute Raum-Zeit-Ausbeute unbefriedigend.

Es wurde nun gefunden, daß man Diketone der Formel I

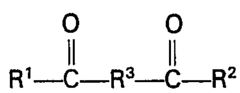

(I),

worin $R^1$ und $R^2$ gleich oder verschieden sein können und jeweils ein Wasserstoffatom oder einen aliphatischen Rest bedeuten, $R^3$ einen, gegebenenfalls durch Reste —O— unterbrochenen, aliphatischen Rest Bezeichnet, darüber hinaus $R^3$, wenn $R^1$ und/oder $R^2$ einen aliphatischen Rest bedeuten, auch für eine Einfachbindung stehen kann, durch Oxidation von Glykolen der Formel II

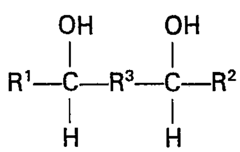

(II),

worin $R^1$, $R^2$ und $R^3$ die vorgenannte Bedeutung besitzen, mit Sauerstoff in Gegenwart eines Kupfer- oder Silberkatalysators vorteilhaft erhält, wenn man Glykole II in Gegenwart eines Katalysators mit 2 oder mehr Schichten Silber- und/oder Kupferkristallen, wobei ein Teil der Schichten 70 bis 95 Gew.-% des Katalysators mit Teilchen der Korngröße 0,75 bis 2,5 mm und der restliche Teil der Schichten 5 bis 30 Gew.-% des Katalysators mit Teilchen der Korngröße 0,1 bis 0,75 mm enthalten, bei einer Temperatur von 450 bis 750°C oxidiert.

Die Umsetzung kann unter Verwendung von Hexandion-2,5 durch die folgenden Formeln wiedergegeben werden:

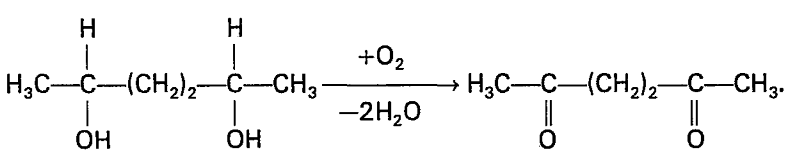

Im Vergleich zu den bekannten Verfahren liefert das Verfahren nach der Erfindung überraschend auf einfacherem und wirtschaftlicherem Wege ein besseres Gesamtergebnis mit Bezug auf Ausbeute, Raum-Zeit-Ausbeute und Reinheit des Endstoffs sowie Lebensdauer des Katalysators. Die Raum-Zeit-Ausbeute ist vergleichsweise besser. Eine umständliche Katalysatorherstellung bzw. Katalysatoraufarbeitung wird vermieden.

Im Hinblick auf die beschriebenen Verfahren besitzt der erfindungsgemäße Katalysator eine höhere Lebensdauer und kann auf einfacherem und wirtschaftlicherem Wege erhalten werden. Silberkristalle aller Teilchengröße, wie sie auch bei der elektrolytischen Herstellung des Silbergranulats anfallen, können verwendet werden. Bei dem erfindungsgemäßen Verfahren werden somit die Elektrolysieranlagen besser ausgenutzt und können entsprechend dimensioniert werden; Energie, Betriebspersonal und Hilfsstoffe, z.B. Salpetersäure, werden eingespart und Operationen wie Wäsche, Siebung und Trocknung des Silbers vereinfacht. Bei den bekannten Verfahren muß man das Silber auf den Träger erst noch aufbringen oder das Silber nachträglich dosieren. Im Falle der Silberspiralen müssen diese erst handwerklich hergestellt, werden. Alle diese vorteilhaften Ergebnisse des erfindungsgemäßen Verfahrens sind überraschend. Man konnte im Hinblick auf den Stand der Technik nicht vermuten, daß durch Verwendung von reinen Silberkristallen besonderer Korngröße anstatt Silberspiralen oder Silber auf Trägern oder Silber mit Promotoren (Phosphor) eine Steigerung der Reaktionsgeschwindigkeit und damit Raum-Zeit-Ausbeute, und noch dazu in wesentlichem Maße, möglich ist.

Bei den erfindungsgemäßen Verfahren handelt es sich im Gegensatz zu dem in DE—C—854 795 beschriebene Verfahren um eine echte Oxidation, die nur in Gegenwart von Sauerstoff abläuft.

Bevorzugte Ausgangsstoffe II und dementsprechend bevorzugte Endstoffe I sind solche, in deren Formeln die Reste $R^1$ und $R^2$ gleich oder verschieden sein können und jeweils ein Wasserstoffatom oder einen Alkylrest mit 1 bis 8 Kohlenstoffatomen bedeuten, $R^3$ einen Alkylenrest mit 1 bis 8 Kohlenstoffatomen, einen Alkenylenrest oder Alkinylenrest mit 2 bis 8 Kohlenstoffatomen bezeichnet, wobei die Kohlenwasserstoffketten der vorgenannten Alkylenreste, Alkenylenreste und Alkinylenreste noch durch 2 Sauerstoffatome oder insbesondere ein Sauerstoffatom unterbrochen werden können, darüber hinaus $R^3$, wenn $R^1$ und/oder $R^2$ einen aliphatischen Rest bedeuten, auch für eine Einfachbindung stehen kann.

Die vorgenannten Reste können noch durch unter den Reaktionsbedingungen inerte Gruppen, z.B. Alkylgruppen mit jeweils 1 bis 4 Kohlenstoffatomen, substituiert sein.

Als geeignete Ausgangsstoffe II kommen z.B. in Frage: 1,2-Propandiol, 1,2-Butandiol, 1,3-Butandiol 1,4-Butandiol, 2,3-Butandiol; 1,2-Pentandiol, 1,3-Pentandiol, 1,4-Pentandiol, 1,5-Pentandiol, 2,3-Pentandiol, 2,4-Pentandiol, 1,2-, 1,3-, 1,4-, 1,5-, 1,6-, 2,3-, 2,4-, 2,5-, 3,4-Hexandiol, 1,2-, 1,3-, 1,4-, 1,5-, 1,6-, 1,7-, 2,3-, 2,4-, 2,5-, 2,6-, 3,4-, 3,5-Heptandiol, 1,2-, 1,3-, 1,4-, 1,5-, 1,6-, 1,7-, 1,8-, 2,3-, 2,4-, 2,5-, 2,6-, 2,7-, 3,4-, 3,5-, 3,6-, 4,5-Octandiol; Diäthylen-, Di-1,3-propylen-, Di-1,3-propylen-, Di-1,2-butylen-, Di-1,3-butylen-, Di-1,4-butylen-glykol; But-2,3-en-1,4-diol, Pent-2,3-en-1,5-diol, Hex-2,3-en-1,6-diol, Hex-3,4-en-1,6-diol, Hept-2,3-en-1,7-diol, Hept-3,4-en-1,7-diol, Oct-2,3-en-1,8-diol, Oct-3,4-en-1,8-diol, Oct-4,5-en-1,8-diol; But-2,3-in-1,4-diol, Pent-2,3-in-1,5-diol, Hex-2,3-in-1,6-diol, Hex-3,4-in-1,6-diol, Hept-2,3-in-1,7-diol, Hept-3,4-in-1,7-diol, Oct-2,3-in-1,8-diol, Oct-3,4-in-1,8-diol, Oct-4,5-in-1,8-diol.

Für das Verfahren geeignete Ausgangsstoffe sind vorteilhaft die Ausgangsstoffe II allein oder gegebenenfalls deren Mischungen mit Wasser oder unter den Reaktionsbedingungen inerten organischen Lösungsmittel; die Konzentration der wäßrigen Gemische kann zweckmäßig zwischen 50 und 90 Gewichtsprozent, die der organischen Gemische zwischen 45 und 90 Geichtsprozent Ausgangsstoff II schwanken. Der Ausgangsstoff II wird in Dampfform, gegebenenfalls im Gemisch mit Wasser- oder Lösungsmitteldampf und gegebenenfalls mit Inertgas, dem Reaktionsraum zugeführt. Als oxidierendes Agens lassen sich sowohl der reine Sauerstoff als auch freien Sauerstoff enthaltende Gase, insbesondere Luft, verwenden. Sauerstoff in Gestalt von Luft, und Ausgangsstoff II werden zweckmäßig im Molverhältnis von 0,3 bis 1,2, insbesondere von 0,4 bis 0,9 Mol Sauerstoff je Mol Ausgangsstoff II angewandt. Die Luft und gegebenenfalls das Inertgas können direkt in die Verdampfung des Ausgangsstoffs II, zweckmäßig in die siedende Ausgangsstoff II/Wasser- oder Lösungsmittel-Mischung, oder an einer beliebigen Stelle vor dem Katalysator eingeleitet werden. Die Verweilzeit im Reaktionsraum beträgt meist höchstens 0,2, zweckmäßig zwischen 0,0005 bis 0,2, vorteilhaft von 0,001 bis 0,2, zweckmäßig von 0,001 bis 0,15, bevorzugt 0,001 bis 0,11 Sekunden. Die Verweilzeit wird auf die Reaktionszone ohne Katalysatorfüllung bezogen und so berechnet. Als Berechnungsgrundlage kann z.B. der Reaktionsraum eines leeren Reaktorrohres dienen.

Die Gesamtschichtdicke des Katalysators beträgt zweckmäßig 10 bis 50, vorzugsweise 15 bis 30 mm. Die Katalysatorteilchen in Gestalt von Silber- und/oder Kupferkristallen befinden sich im Katalysator des zweckmäßig vertikal aufgestellten Reaktors je nach Korngröße in einem oberen und unteren Teil der Gesamtschicht angeordnet. Das gesamte Katalysatorbett liegt zweckmäßig auf einem Netz aus Silber oder Edelstahl (vorgeglüht). Das Ausgangsgemisch aus dampfförmigem Ausgangsstoff II und Sauerstoff bzw. Luft wird im allgemeinen von oben nach unten geführt, so daß die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäß alle Angaben der Beschreibung über oberen (unteren) Teil des Katalysators für den entsprechenden, dem Ausgangsgemisch (dem abgeführten Reaktionsgemisch) zugewandten Teil, z.B. bei horizontal angeordneten Reaktoren für den vorderen (hinteren) Teil des Katalysators. Im unteren Teil befinden sich 70 bis 95, vorzugsweise 80 bis 90 Gewichtsprozent aller Katalysatorteilchen und im oberen Teil 5 bis 30, vorzugsweise 10 bis 20 Gewischtsprozent aller Katalysatorteilchen. Die Teilchen des unteren Schichtteils haben Korngrößen von 0,75 bis 2,5 die des oberen Schichtteils 0,1 bis 0,75 mm. Jeder Schichtteil kann aus einer oder mehreren Schichten, vorzugsweise aus 1, 2 oder 3 Schichten bestehen. Bevorzugt ist ein 3- bis 7-Schichtenkatalysator, insbesondere ein 3-oder 4-Schichtenkatalysator. Jede dieser Schichten unterscheidet sich von der anderen in der Korngröße der Silber- und/oder Kupferkristalle und meistens auch im zugehörigen Gewichtsanteil des Gesamtkatalysators.

Hat der obere Schichtteil 2 Schichten, so haben seine obere Schicht bevorzugt einen Anteil von 0,5 bis 29,5 Gewichtsprozent und Teilchen einer Korngröße von 0,1 bis 0,4 mm und seine untere Schicht entsprechend einen Gewichtsanteil von 0,5 bis 29,5 Gewichtsprozent und Teilchen der Korngröße von 0,4 bis 0,75 mm. Sind 3 Schichten im oberen Schichtteil vorhanden, so sind mit Bezug auf Gewichtsanteil am Gesamtkatalysator (Korngröße der Teilchen) bevorzugt: obere Schicht 0,5 bis 29 (0,1 bis 0,4 mm); mittlere Schicht 0,5 bis 29 (0,4 bis 0,6 mm); untere Schicht 0,5 bis 29 (0,6 bis 0,75 mm) Gewichtsprozent. Entsprechend sind bei dem unteren Schichtteil mit Bezug auf Gewichtsanteil (Korngröße der Teilchen) bevorzugt:

a) 2 Schichten: obere Schicht 5—90 (0,75—1 mm) Gew.%;
untere Schicht 5—90 (1,00—2,5 mm) Gew.%.
b) 3 Schichten: obere Schicht 5—85 (0,75—1 mm) Gew.%;
mittlere Schicht 5—85 (1—1,5 mm) Gew.%;
untere Schicht 5—85 (1,5—2,5 mm) Gew.%.

Die Schichtung jeder einzelnen Schicht ist meist regelmäßig, so daß die Schichtdicke der Einzelschicht über den ganzen Schichtquerschnitt hinweg gleich ist. In diesen Fällen hängt die Schichtdicke direkt von den vorgenannten Gewichtsanteilen Gesamtkatalysator und der jeweiligen Korngröße der Teilchen ab. Man Kann aber auch eine unregelmäßige Schichtung aller oder mehrerer oder zweckmäßig einer Schicht vornehmen, z.B. in der Mitte, auf den Seiten oder vorteilhaft am Rande der Schicht die Hauptmenge der Katalysatorteilchen aufgeben und entsprechend nur eine kleinere Restmenge auf die übrige Schicht verteilen.

Ein besonders vorteilhafter Katalysator hat die folgende Zusammensetzung:

Schicht 1 (oberste): 5—30 Gew.% des Katalysators mit Teilchen der Krongröße 0,1—0,75 mm
Schicht 2: 5—90 Gew.% des Katalysators mit Teilchen der Korngröße 0,75—1 mm
Schicht 3 (unterste): 5—90 Gew.% des Katalysators mit Teilchen der Korngröße 1—2,5 mm.

Zweckmäßig belastet man den Katalysator mit 0,2 bis 3 t, insbesondere 0,4 bis 1,6 t dampfförmigem Ausgangsstoff II je $m^2$ Katalysatorbettquerschnitt und Stunde. Zur großtechnischen Ausführung verwendet man bevorzugt Katalysatorbettdurchmesser von mindestens 0,1, zweckmäßig 0,2 bis 3 Metern. Bevorzugt sind Gemische von Silber und Kupfer oder insbesondere Silberkristalle allein.

Die Umsetzung wird bei einer Temperatur von 450 bis 750°C, vorzugsweise bei einer Temperatur von 500 bis 700°C, insbesondere 600 bis 700°C, drucklos oder unter Druck, im allgemeinen bei Drücken zwischen 0,8 und 2 bar, vorzugsweise zwischen 0,8 und 1,8 bar, insbesondere 1,05 bis 1,5 bar, diskontinuierlich oder vorzugsweise kontinuierlich durchgeführt.

Die Oxidation kann wie folgt durchgeführt werden: In ein Verdampfungsaggregat, z.B. einen Fallstromverdampfer, werden einzeln oder im Gemisch Ausgangsstoff II und gegebenenfalls Wasser eingegeben und verdampft. Anschließend wird das Gasgemisch aus dampfförmigem Ausgangsstoff II, Luft, gegebenenfalls Inertgas und Wasserdampf in vorgenannten Mengen bei der Reaktionstemperatur durch den Katalysator geleitet. Es ist vorteilhaft, die die Katalysatorzone verlassenden Reaktionsgase innerhalb kurzer Zeit abzukühlen, z.B., auf Temperaturen von 20 bis 160°C. Der Hauptteil des Endstoffs I wird so kondensiert. Das abgekühlte Gasgemisch wird dann zweckmäßig einem Absorptionsturm zugeführt, in welchem der Endstoff I mit einem geeigneten Lösungsmittel wie Dimethylformamid, Dimethylsulfoxid, Aceton, Methanol oder Wasser sowie deren Gemische und/oder in vorgelegtem Kondensat früherer Umsetzungen, vorteilhaft im Gegenstrom, aus dem Gasgemisch gewaschen wird. Aus Kondensat und den Absorbaten wird der Endstoff I dann in üblicher Weise, z.B. durch Destillation, isoliert.

Die nach dem Verfahren der Erfindung herstellbaren Endstoffe I sind wertvolle Ausgangsstoffe für die Herstellung von Farbstoffen, Pharmazeutika, Kunststoffen, Riechstoffen, Knitterfestmitteln, Hilfsmitteln zur Erhöhung der Reißfestigkeit und Elastizität von Faserstoffen, Härtern in der photographischen Industrie und Textilhilfsmitteln zur Verhinderung des Einlaufens nach dem Waschen, Papierhilfsmitteln, z.B. zur Erhöhung der Naßfestigkeit.

Die in den folgenden Beispieln angeführten Teile bedeuten Gewichtsteile.

Beispiel 1

Man verwendet eine Anlage mit Verdampfer und einem senkrechten Rohrreaktor. Der Reaktor

enthält an seinem Kopf die Zuführung für das dampfförmige Ausgangsgemisch und die Reaktorhaube. Die Katalysatorschicht liegt unterhalb des Reaktorkopfes, weiter unten folgt eine Kühlzone. Der Reaktor ist mit einer Absorptionskolonne verbunden.

In den Reaktor wird ein Katalysator aus Silberkristallen (28 Teilen) folgender Zusammensetzung eingetragen (Schichthöhe 20 mm):

| | Anteil am Katalysator (Gew.%) | Korngröße (mm) |
|---|---|---|
| Schicht 1 | 14,1 | 0,1—0,75 |
| Schicht 2 | 5,9 | 0,75—1 |
| Schicht 3 | 80 | 1—2,5 |

Dem Verdampfer wird pro Stunde ein Gemisch von 254 Teilen n-Hexandiol-2,5 und 247 Teile Luft zugeführt und verdampft. Das dampfförmige Ausgangsgemisch wird durch den Katalysator geleitet und bei 650°C und 1,4 bar umgesetzt. Die Belastung beträgt 0,8 $t/m^2 \cdot h$. Die Verweilzeit, bezogen auf das leere Rohr, beträgt 0,1 Sekunde. Das gasförmige Reaktionsgemisch wird nun auf 20°C abgekühlt und anschließend mit Wasser gewaschen. In Form einer 49,6-gewichtsprozentigen Lösung erhält man 210,3 Teile pro Stunde n-Hexandion-2,5 vom Kp 191°C, entsprechend einer Ausbeute von 84,2% der Theorie, keinen Anteil an unumgesetztem n-Hexandiol-2,5 sowie 6,8 Teile Hexanol-2-on-5. Die Raum-Zeit-Ausbeute beträgt 33,4 $g/cm^3 \cdot h$. Die Lebensdauer des Katalysators beträgt 125 Tage, der Umsatz beträgt 100 Prozent.

Beispiel 2

Es wird die gleiche Anlage wie in Beispiel 1 verwendet. In den Reaktor wird ein Katalysator aus Silberkristallen (28 Teilen) folgender Zusammensetzung eingetragen (Schichthöhe 20 mm):

| | Anteil am Katalysator (Gew.%) | Korngröße (mm) |
|---|---|---|
| Schicht 1 | 18,5 | 0,1—0,75 |
| Schicht 2 | 81,5 | 0,75—2,5 |

Dem Verdampfer wird pro Stunde ein Gemisch von 314 Teilen n-Hexandiol-2,5 und 212 Teilen Luft zugeführt und verdampft. Das dampfförmige Ausgangsgemisch wird durch den Katalysator geleitet und bei 600°C und 1,4 bar umgesetzt. Die Belastung beträgt 1 $t/m^2 \cdot h$. Die Verweilzeit, bezogen auf das leere Rohr, beträgt 0,1 Sekunde. Das gasförmige Reaktionsgemisch wird nun auf 20°C abgekühlt und mit Wasser gewaschen. In Form einer 50,8 gewichtsprozentigen Lösung erhält man 250 Teile pro Stunde n-Hexandion-2,5 vom Kp 191°C, entsprechend einer Ausbeute von 81 Prozent der Theorie, 4 Teile pro Stunde unumgesetzter n-Hexandiol-2,5 sowie 17,7 Teile Hexanol-2-on-5. Die Raum-Zeit-Ausbeute beträgt 39,8 $g/cm^3 \cdot h$. Die Lebensdauer des Katalysators beträgt 130 Tage, der Umsatz beträgt 98,7 Prozent.

Beispiel 3

Analog Beispiel 1 wird die Umsetzung mit 500 Teilen pro Stunde 2,3-Butandiol und 460 Teilen Luft bei 640°C und 1,1 bar durchgeführt. Die Belastung beträgt 1,6 $t/m^2 \cdot h$. Man erhält 361 Teile pro Stunde 2,3-Butandion, entsprechend einer Ausbeute von 76% der Theorie, neben 35 Teilen pro Stunde Butan-3-ol-2-on und 25 Teilen pro Stunde unumgesetztem 2,3-Butandiol. Die Lebensdauer des Katalysators beträgt 125 Tage, der Umsatz 95 Prozent. Die Raum-Zeit-Ausbeute beträgt 57 $g/cm^3 \cdot h$.

Beispiel 4 (Vergleichsbeispiel nach DE—C—854 795)

Über 1 l eines wie im Beispiel dieser Patentschrift hergestellten Kupferkatalysators führt man stündlich bei 300°C 500 Teile 2,3-Butandiol. Man erhält stündlich als Hauptprodukt 367 Teile Butan-3-ol-on (Acetoin), entsprechend einer Ausbeute von 75% der Theorie. Daneben entstehen stündlich 38 Teile 2,3-Butandiol (Diacetyl), entsprechend einer Ausbeute von 8% der Theorie. Daneben werden 75 Teile an unumgesetztem 2,3-Butanediol erhalten. Der Umsatz beträgt 85%.

Das Hauptprodukt nach der Lehre dieser Patentschrift ist also mit einer Ausbeute von 75% der Ketoalkohol Acetoin, d.h. die nur an einer der beiden Alkoholfunktionen dehydrierte Verbindung.

Das beim erfindungsgemäßen Verfahren angestrebte und—wie im Beispiel 3 gezeigt—mit einer Ausbeute von 76% der Theorie hergestellte Diketon Diacetyl fällt bei dem Vergleichsbeispiel lediglich mit einer Ausbeute von 8% der Theorie an.

Das erfindungsgemäße Verfahren zur Herstellung von Diketonen durch Oxidation mit Sauerstoff an kristallinem Silber und/oder Kupfer in der erfindungsgemäßen Schichtenstruktur und Korngrößen bestimmter Menge liefert damit überraschend weitaus bessere Ausbeuten an der zweifach dehydrierten Verbindung als das Verfahren nach der DE—C—854 795.

Die Raum-Zeit-Ausbeute an dem erfindungsgemäßen 2,3-Butandion ist mit 57 g/cm³ · h nach dem erfindungsgemäßen Verfahren um Größenordnungen höher als im Vergleichsbeispiel mit 0,038 g/cm³ · h für 2,3-Butandion bzw. mit 0,367 g/cm³ · h für Butan-3-ol-2-on.

Beispiel 5 (Vergleichsbeispiel nach US—A—2 051 266)

Als Vergleichsbeispiel wurde hier das in der Patentschrift beschriebene Beispiel (Seite 3, rechte Spalte, Zeilen 51 bis 61) nachvollzogen.

Über 1 l eines nach der Lehre der Patentschrift hergestellten Kupferkatalysator werden stündlich 500 Teile 2,3-Butandiol zusammen mit 685 Teilen Luft bei einer Katalysatortemperatur von 270 bis 275°C geführt.

Man erhält nach Abkühlung der Reaktionsgase auf 20°C eine flüssige Produktmischung aus 2,3-Butandion (Diacetyl), Butan-3-ol-2-on (Acetoin), unumgesetztem 2,3-Butandiol und Zersetzungsprodukten sowie einen Abgasstrom, der Kohlendioxid und Kohlenmonoxid enthält. Die Zusammensetzung der flüssigen Phase entspricht dabei dem Patentbeispiel.

Im einzelnen werden stündlich 180 Teile 2,3-Butandion, entsprechend einer Ausbeute von 38% der Theorie, sowie 155 Teile Butan-3-ol-2-on, entsprechend einer Ausbeute von 32% der Theorie, erhalten. An unumgesetztem 2,3-Butandiol werden stündlich 26 Teile erhalten; der Umsatz beträgt damit 95%.

Im Vergleich zu diesem Verfahren der US—A—2 051 266 liefert das erfindungsgemäße Verfahren die ca. doppelte Ausbeute an dem gewünschten Diketon in deutlich höhere Reinheit des Reaktionsaustrages.

Die Raum-Zeit-Ausbeute bei unserem Verfahren liegt mit 57 g/cm³ h um Größenordnungen höher als die im Vergleichsbeispiel mit 0180 g/cm³ · h für 2,3-Butandion.

## Patentanspruch

Verfahren zur Herstellung von Diketonen der Formel I

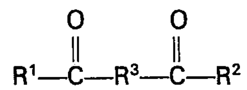 (I),

worin $R^1$ und $R^2$ gleich oder verschieden sein können und jeweils ein Wasserstoffatom oder einen aliphatischen Rest bedeuten, $R^3$ einen, gegebenenfalls durch Reste —O— unterbrochenen, aliphatischen Rest bezeichnet, darüber hinaus $R^3$, wenn $R^1$ und/oder $R^2$ einen aliphatischen Rest bedeuten, auch für eine Einfachbindung stehen kann, durch Oxidation von Glykolen der Formel II

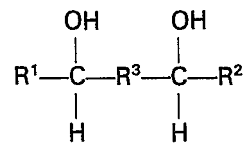 (II),

worin $R^1$, $R^2$ und $R^3$ die vorgenannte Bedeutung besitzen, mit Sauerstoff in Gegenwart eines Kupfer- oder Silberkatalysators, dadurch gekennzeichnet, daß man Glykole II in Gegenwart eines Katalysators mit 2 oder mehr Schichten Silber- und/oder Kupferkristallen, wobei ein Teil der Schichten 70 bis 95 Gew.-% des Katalysators mit Teilchen der Korngröße 0,75 bis 2,5 mm und der restliche Teil der Schichten 5 bis 30 Gew.-% des Katalysator mit Teilchen der Krongröße 0,1 bis 0,75 mm enthalten, bei einer Temperatur von 450 bis 750°C oxidiert.

## Claim

A process for the preparation of a diketone of the formula I

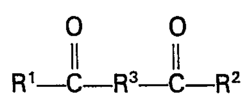 (I),

where $R^1$ and $R^2$ may be identical or different and each is hydrogen or an aliphatic radical, $R^3$ is an aliphatic radical which may or may not be interrupted by —O— radicals and, if $R^1$ and/or $R^2$ is an aliphatic radical, may also be a single bond, by oxidation of a glycol of the formula II

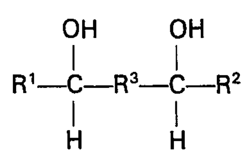 (II),

where $R^1$, $R^2$ and $R^3$ have the above meanings, with oxygen in the presence of a copper or silver catalyst, characterized in that a glycol II is oxidized at from 450 to 750°C in the presence of a catalyst comprising 2 or more layers of silver crystals and/or copper crystals, some of the layers, accounting for from 70 to 95 percent by weight of the catalyst, containing particles having a size of from 0.75 to 2.5 mm, and the remainder of the layers, accounting for from 5 to 30 percent by weight of the catalyst, containing particles having a size of from 0.1 to 0.75 mm.

**Revendication**

Procédé de préparation de dicétones de la formule I

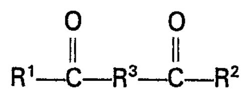

(I),

dans laquelle $R^1$ et $R^2$, qui peuvent être identiques ou différents, désignent chacun un atome d'hydrogène ou un reste aliphatique et $R^3$ désigne un reste aliphatique éventuellement interrompu par des groupes —O— ou représente une liaison simple, lorsque $R^1$ et(ou) $R^2$ désignent un reste aliphatique, par oxydation de glycols de la formule II

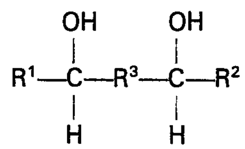

(II),

dans laquelle $R^1$, $R^2$ et $R^3$ possèdent les significations définies, par l'oxygène en présence d'un catalyseur au cuivre ou à l'argent, caractérisé en ce que l'oxydation de glycols II est effectuée à une température de 450 à 750°C en présence d'un catalyseur constitué de deux ou de plus de deux couches de cristaux d'argent et(ou) de cuivre, une partie de ces couches contenant 70 à 95% du poids du catalyseur sous forme de particules d'une granulométrie de 0,75 à 2,5 mm et la partie restante de ces couches contenant 5 à 30% du poids du catalyseur sous forme de particules d'une granulométrie de 0,1 à 0,75 mm.